# FASCICULE DE BREVET EUROPEEN

(11) **EP 0 866 704 B1**
(45) Date de publication et mention de la délivrance du brevet: **11.04.2001**
(21) Numéro de dépôt: 96934975.2
(22) Date de dépôt: 25.10.1996
(51) Int. Cl.: A61K 31/44, A61K 38/30, A61K 38/18

(54) **UTILISATION DE LA 1-(2-NAPHT-2-YLETHYL)-4-(3-TRIFLUOROMETHYLPHENYL)-1,2,3,6-TETRAHYDROPYRIDINE POUR LA PREPARATION DE MEDICAMENTS DESTINES AU TRAITEMENT DE LA SCLEROSE LATERALE AMYOTROPHIQUE**
VERWENDUNG VON 1-(2-NAPHTYLETHYL)-4-(3-TRIFLUOROMETHYLPHENYL)-1,2,3,6-TETRAHYDROPYRIDIN ZUR HERSTELLUNG EINES MEDIKAMENTS ZUR BEHANDLUNG DER AMYOTROPHEN LATERALSKLERODE
USE OF 1-(2-NAPHTH-2-YLETHYL)-4-(3-TRIFLUOROMETHYLPHENYL)-1,2,3,6-TETRAHYDROPYRIDINE FOR PREPARING DRUGS FOR TREATING AMYOTROPHIC LATERAL SCLEROSIS

(30) Priorité: 26.10.1995 FR 9512635; 13.06.1996 FR 9607336
(43) Date de publication de la demande: 30.09.1998
(73) Titulaire: SANOFI-SYNTHELABO, 75013 Paris (FR)
(72) Inventeur: DOUILLET, Patrice, F-34980 St.-Gély-du-Fesc (FR); FOURNIER, Jacqueline, F-31830 Plaisance-du-Touch (FR)
(74) Mandataire: Gillard, Marie-Louise
(86) Numéro de dépôt international: FR9601674
(87) Numéro de publication internationale: WO9715304

(56) Documents cités:
- EP-A- 0 458 696
- EP-A- 0 498 718
- EP-A- 0 558 861
- XX CONGRESS OF THE SPANISH SOCIETY OF PHARMACOLOGY, Septembre 1996, GRANADA, page 206 XP000645694 L. LACOMBLEZ ET AL.: "SAFETY AND EFFICACY CONTROLLED TRIAL OF SR 57746A IN AMYOTROPHIC LATERAL SCLEROSIS"
- ANNALS OF NEUROLOGY, vol. 38, no. 6, 1995, page 971 XP002027393 W.G. BRADLEY ET AL.; E.C. LAI ET AL.: "1.A PHASE I/II STUDY OF RECOMBINANT HUMAN BRAIN-DERIVED NEUROTROPHIC FACTOR IN PATIENTS WITH AMYOTROPHIC LATERAL SCLEROSIS; 2.ADOUBLE-BLIND, PLACEBO-CONTROLLED STUDY OF RECOMBINANT HUMAN INSULIN-LIKE GROWTH FACTOR 1 IN THE TREATMENT OF AMYOTROPHIC LATERAL SCLEROSIS" cité dans la demande
- NATURE, vol. 360, 1992, pages 753-759, XP000645418 Q. YAN ET AL.: "BRAIN-DERIVED NEUROTROPHIC FACTOR RESCUES SPINAL MOTOR NEURONS FROM AXOTOMY-INDUCED CELL DEATH" cité dans la demande
- NEUROSCIENCE, vol. 55, no. 3, 1993, pages 629-641, XP002008237 J. FOURNIER ET AL.: "PROTECTIVE EFFECTS OF SR 57746A IN CENTRAL AND PERIPHERAL MODELS OF NEURODEGENERATIVE DISORDERS IN RODENTS AND PRIMATES"
- FUNDAMENTAL & CLINICAL PHARMACOLOGY, vol. 7, no. 7, 1993, page 359 XP002008238 T. GAUTHIER ET AL.: "SR 57746A: INDUCTION OF NGF GENE EXPRESSION AND SYNTHESIS IN ASTROCYTOMA AND FIBROBLAST CELL LINES AND BENEFICIAL EFFECTS IN A PERIPHERAL DEGENERATIVE MODEL IN RATS"
- JOURNAL OF NEUROBIOLOGY, vol. 25, no. 11, 1994, pages 1418-1435, XP000575086 F. HEFTI: "NEUROTROPHIC FACTOR THERAPY FOR NERVOUS SYSTEM DEGENERATIVE DISEASES"
- CURRENT OPINION IN NEUROBIOLOGY, vol. 4, no. 5, 1994, pages 752-757, XP002008239 O. LINDVALL ET AL.: "CLINICAL APPLICTION OF CELL TRANSPLANTATION AND NEUROTROPHIC FACTORS IN CNS DISORDERS"
- EXPERIMENTAL NEUROLOGY, vol. 124, no. 1, 1993, pages 64-72, XP002008240 J.L. SEEBURGER ET AL.: "EXPERIMENTAL RATIONALE FOR THE THERAPEUTIC USE OF NEUROTROPHINS IN AMYOTROPHIC LATERAL SCLEROSIS"

## Description

La présente invention concerne l'utilisation de la 1-(2-napht-2-yléthyl)-4-(3-trifluorométhylphényl)-1,2,3,6-tétrahydropyridine ou de ses sels d'addition avec des acides pharmaceutiquement acceptables pour la préparation de médicaments destinés au traitement de la sclérose latérale amyotrophique (SLA).

La SLA est une grave maladie du motoneurone à évolution progressive qui conduit à l'atrophie musculaire et évolue le plus souvent en quelques années en une insuffisance respiratoire fatale.

Très peu de produits sont à l'étude pour la SLA notamment des composés peptidiques, tels que l'IGF-1 (de l'anglais Insulin-like Growth Factor 1), le BDNF (de l'anglais Brain Derived Neurotrophic Factor) ou le CNTF (de l'anglais Ciliary Neurotrophic Factor) décrits dans Annals of Neurology, 1995, 38:971, Nature, 1992, 360:753-759, J. Neurobiol. 1994, 25(11):1418-1435, Current Opinion in Neurobiology 1994, 4(5) : 752-757 ou Experimental Neurology 1993, 124:64-72.

Le seul composé non-peptidique qui a été testé dans cette maladie est le riluzole dont le nom chimique est 2-amino-6-trifluorométhoxybenzothiazole, qui serait apparemment en mesure de ralentir la progression de la maladie chez un groupe particulier de sujets atteints de SLA (G. Bensimon et al., N. Engl. J. Med., 1994, 330:585-591; Scrip, 1995, No 2035:21), mais aucun produit efficace dans le traitement de cette maladie n'est actuellement disponible sur le marché pharmaceutique. Selon l'article de G. Bensimon et al. cité ci-dessus, le riluzole prolonge la survie des patients atteints de SLA, mais les effets secondaires tels que l'asthénie, la spasticité et l'augmentation des taux de transaminases, détériorent la qualité de la vie desdits patients.

EP-A-458696 décrit l'utilisation de la 1-(2-napht-2-yléthyl)-4-(3-trifluorométhylphényl)-1,2,3,6-tétrahydropyridine, denommée en littérature SR 57746, pour la préparation de médicaments destinés à combattre des états neurodégéneratifs. Dans ce document, l'activité neurotrophique du SR 57746 est indiquée comme utile dans les troubles de la mémoire, la démence vasculaire, les troubles post-encéphalitiques, les troubles post-apoplectiques, les syndrômes post-traumatiques dûs à un traumatisme crânien, les troubles dérivant d'anoxies cérébrales, la maladie d'Alzheimer, la démence sénile, la démence subcorticale, telle que la chorée de Huntington et la maladie de Parkinson, la démence provoquée par le SIDA, les neuropathies dérivées de morbidité ou dommage des nerfs sympathiques ou sensoriels, les maladies cérébrales telles que l'oedème cérébral, et les dégénérescences spinocérébelleuses.

EP-A-498718 décrit l'utilisation de dérivés de 4-(3-trifluorométhylphényl)-1,2,3,6-tétrahydropyridine comme capteurs de radicaux libres.

L'action neurotrophique du SR 57746 sur le système nerveux est semblable à celle du NGF, de l'anglais Nerve Growth Factor (EP-A-458696) et il est notamment réputé assurer une neuroprotection en induisant les effets du système NGF (J. Fournier, Neuroscience, 1993, 55(3):629-641).

Il a été maintenant trouvé que l'administration de SR 57746 ou de l'un de ses sels d'addition avec des acides pharmaceutiquement acceptables, ralentit significativement la progression de la SLA tout en améliorant également la qualité de vie des patients. Cette action thérapeutique du SR 57746 n'est pas liée à la libération du NGF car ce dernier n'est pas réputé être un facteur trophique pour les motoneurones, comme indiqué par exemple dans Neuron, 1988, 1(4):335-43; J. Comp. Neurol., 1982, 210/2:174-189; et Eur. J. Neurosci., 1993, 5(5):466-474.

L'activité du SR 57746 dans le traitement de la SLA est donc inattendue.

Ainsi la présente invention a pour objet, selon un de ses aspects, l'utilisation de la 1-(2-napht-2-yléthyl)-4-(3-trifluorométhylphényl)-1,2,3,6-tétrahydropyridine ou de l'un de ses sels d'addition avec des acides pharmaceutiquement acceptables pour la préparation de médicaments destinés au traitement de la sclérose latérale amyotrophique.

L'activité clinique du SR 57746 dans cette maladie a été mise en évidence par une étude conduite pour évaluer les indices d'activité sur les signes cliniques et fonctionnels de la SLA et pour évaluer la tolérance après administration prolongée du chlorhydrate de 1-(2-napht-2-yléthyl)-4-(3-trifluorométhylphényl)-1,2,3,6-tétrahydropyridine (SR 57746 A).

Dans cette étude clinique, 54 patients ont été randomisés en double aveugle pour recevoir le SR 57746 A par voie orale à une dose correspondant à 2 mg/jour de base libre, pendant une durée de 8 mois. Les doses indiquées dans la présente description se réfèrent toujours à la quantité de base libre administrée ou contenue dans l'unité de dosage.

Plusieurs variables ont été prises en considération pour évaluer ladite activité du SR 57746 A dans le traitement de la SLA, notamment:
- un test sur la capacité vitale par lequel on mesure la capacité respiratoire maximale ;
- un test musculaire par lequel on évalue la force musculaire ;
- un examen neurologique par lequel on évalue les réflexes ;
- les examens de Norris (bulbaire et membres), par lesquels on évalue la capacité des patients à effectuer certains mouvements.

L'évaluation de l'efficacité du composé en examen est exprimée en scores qui indiquent l'état de progression de la maladie chez le patient par rapport aux sujets sains.

Les résultats obtenus montrent que les deux groupes de traitement (2 mg/jour de SR 57746 A et placébo) se différencient progressivement au cours des 8 mois, de façon remarquable pour les trois variables principales étudiées: capacité vitale respiratoire, force musculaire et échelles fonctionnelles de Norris.

Les moyennes des pentes évolutives des patients traités pendant 8 mois par le SR 57746 A sont de l'ordre de 40% plus faibles que celles des patients traités par placébo.

Les résultats obtenus par cette étude montrent que le SR 57746 A est en mesure de ralentir de manière significative la progression de la SLA. Le produit est également très bien toléré comme il a été constaté pendant les 8 mois de traitement.

Selon la présente invention, le SR 57746 peut être administré seul ou en association avec d'autres principes actifs, notamment avec le riluzole en thérapie combinée ou dans la même forme pharmaceutique.

Plus particulièrement, la présente invention concerne l'utilisation de la 1-(2-napht-2-yléthyl)-4-(3-trifluorométhylphényl)-1,2,3,6-tétrahydropyridine et du 2-amino-6-trifluorométhoxybenzothiazole ou de leurs sels d'addition avec des acides pharmaceutiquement acceptables pour la préparation d'un médicament destiné au traitement de la sclérose latérale amyotrophique.

Les compositions pharmaceutiques contenant l'association de SR 57746 et de riluzole ou de leurs sels pharmaceutiquement acceptables et l'utilisation de ladite association pour la préparation de médicaments destinés au traitement de la sclérose latérale amyotrophique, constituent des aspects ultérieurs de la présente invention.

Ainsi, la présente invention concerne aussi une composition pharmaceutique contenant en tant que principes actifs la 1-(2-napht-2-yléthyl)-4-(3-trifluorométhylphényl)-1,2,3,6-tétrahydropyridine et le 2-amino-6-trifluorométhoxybenzothiazole ou leurs sels d'addition avec des acides pharmaceutiquement acceptables.

Selon un autre aspect, l'invention concerne également une trousse destinée au traitement de la SLA qui contient :
(a) une ou plusieurs doses unitaires de 1-(2-napht-2-yléthyl)-4-(3-trifluorométhylphényl)-1,2,3,6-tétrahydropyridine ou d'un de ses sels pharmaceutiquement acceptables ; et
(b) une ou plusieurs doses unitaires de 2-amino-6-trifluorométhoxybenzothiazole, ou d'un de ses sels pharmaceutiquement acceptables ; ladite trousse étant destinée à l'administration simultanée, séquentielle ou étalée dans le temps des composants (a) et (b).

Le SR 57746 et ses sels d'addition avec des acides pharmaceutiquement acceptables sont de préférence administrés par voie orale.

Dans les compositions pharmaceutiques de la présente invention pour l'administration orale, le SR 57746 ou un sel pharmaceutiquement acceptable de celui-ci utilisé comme principe actif, peut être administré sous forme unitaire d'administration, en mélange avec des supports pharmaceutiques classiques, aux animaux et aux êtres humains pour le traitement des affections susdites. Les formes unitaires d'administration appropriées comprennent par exemple les comprimés éventuellement sécables, les gélules, les poudres, les granules et les solutions ou suspensions orales.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

La quantité de principe actif à administrer dépend comme toujours du dégré d'avancement de la maladie ainsi que de l'age et du poids du patient. Néanmoins, les doses unitaires comprennent généralement de 0,5 à 10 mg, avantageusement de 1 à 5, de préférence de 1 à 3 mg, par exemple 1 - 1,5 - 2 - 2,5 - 3 mg de principe actif. Ces doses unitaires sont administrées normalement une ou plusieurs fois par jour, de préférence une à trois fois par jour, la dose globale chez l'homme étant variable entre 0,5 et 50 mg par jour, par exemple de 1 à 20 mg par jour.

Lorsque on administre le SR 57746 en association avec d'autres principes actifs, notamment avec le riluzole, les doses sont choisies parmi les doses qui seraient administrées pour chaque médicament, selon la gravité de la maladie, l'age et du poids du patient. Des associations avantageuses contiennent de 0,5 à 10 mg de SR 57746 ou d'un de ses sels pharmaceutiquement acceptables et de 30 à 100 mg de riluzole ou d'un de ses sels pharmaceutiquement acceptables par unité de dosage, celles contenant 0,5 mg de SR 57746 ou d'un de ses sels pharmaceutiquement acceptables et 50 mg de riluzole ou d'un de ses sels pharmaceutiquement acceptables, 1 mg de SR 57746 ou d'un de ses sels pharmaceutiquement acceptables et 50 mg de riluzole ou d'un de ses sels pharmaceutiquement acceptables, 1,5 mg de SR 57746 ou d'un de ses sels pharmaceutiquement acceptables et 50 mg de riluzole ou d'un de ses sels pharmaceutiquement acceptables, 2 mg de SR 57746 ou d'un de ses sels pharmaceutiquement acceptables et 50 mg de riluzole ou d'un de ses sels pharmaceutiquement acceptables, par unité de dosage, en mélange avec un excipient pharmaceutique, étant particulièrement préférées. Ces unités de dosage peuvent être administrées 1 ou 2 fois par jour.

### EXEMPLES 1 à 3

On prépare des gélules contenant 0,5 mg, 1 mg, et 2 mg, de SR 57746 (préparé comme décrit dans EP-A-0 101 381).

Le produit SR 57746 A est tamisé à 0,200 mm puis prémélangé avec les excipients. Ce mélange est tamisé à 0,315 mm, rémélangé, puis à nouveau tamisé à 0,315 mm. Après un dernier mélange, le remplissage des gélules est effectué.

| | | | |
|---|---|---|---|
| SR 57746 A | 0,548 mg | 1,096 mg | 2,192 mg |
| Amidon de maïs modifié | 142,852 mg | 142,304 mg | 141,208 mg |
| Cellulose microcristalline | 26,0 mg | 26,0 mg | 26,0 mg |
| Silice colloïdale anhydre | 0,20 mg | 0,20 mg | 0,20 mg |
| Stéarate de magnésium | 0,40 mg | 0,40 mg | 0,40 mg |

## Revendications

1. Utilisation de la 1-(2-napht-2-yléthyl)-4-(3-trifluorométhylphényl)-1,2,3,6-tétrahydropyridine ou de l'un de ses sels d'addition avec des acides pharmaceutiquement acceptables pour la préparation de médicaments destinés au traitement de la sclérose latérale amyotrophique.

2. Utilisation selon la revendication 1, où le sel d'addition d'acide est le chlorhydrate.

3. Utilisation selon la revendication 1 ou 2, où le médicament est une composition pharmaceutique sous forme de dosage unitaire.

4. Utilisation selon la revendication 3, dans laquelle la forme de dosage unitaire comprend de 0,5 à 10 mg de principe actif.

5. Utilisation selon la revendication 4, dans laquelle la forme de dosage unitaire comprend de 1 à 5 mg de principe actif.

6. Utilisation selon l'une quelconque des revendications 3 à 5, où la composition pharmaceutique est sous forme d'une composition pour l'administration orale.

7. Utilisation de la 1-(2-napht-2-yléthyl)-4-(3-trifluorométhylphényl)-1,2,3,6-tétrahydropyridine et du 2-amino-6-trifluorométhoxybenzothiazole ou de leurs sels d'addition avec des acides pharmaceutiquement acceptables pour la préparation d'un médicament destiné au traitement de la sclérose latérale amyotrophique.

8. Composition pharmaceutique contenant en tant que principes actifs la 1-(2-napht-2-yléthyl)-4-(3-trifluorométhylphényl)-1,2,3,6-tétrahydropyridine et le 2-amino-6-trifluorométhoxybenzothiazole ou leurs sels d'addition avec des acides pharmaceutiquement acceptables.

9. Trousse destinée au traitement de la sclérose latérale amyotrophique contenant :
a) une ou plusieurs doses unitaires de 1-(2-napht-2-yl)-4-(3-trifluorométhylphényl)-1,2,3,6-tétrahydropyridine ou de l'un de ses sels pharmaceutiquement acceptables, et
b) une ou plusieurs doses unitaires de 2-amino-6-trifluorométhoxybenzothiazole ou de l'un de ses sels pharmaceutiquement acceptables.

## Claims

1. Use of 1-(2-naphth-2-ylethyl)-4-(3-trifluoromethylphenyl)-1,2,3,6-tetrahydropyridine or one of its addition salts with pharmaceutically acceptable acids for the preparation of medicaments intended for the treatment of amyotrophic lateral sclerosis.

2. Use according to claim 1 wherein the acid addition salt is the hydrochloride.

3. Use according to claim 1 or 2 wherein the medicament is a pharmaceutical composition in the form of a unit dosage.

4. Use according to claim 3 wherein the unit dosage form comprises from 0.5 to 10 mg of active principle.

5. Use according to claim 4 wherein the unit dosage form comprises from 1 to 5 mg of active principle.

6. Use according to any one of claims 3 to 5 wherein the pharmaceutical composition is in the form of a composition for oral administration.

7. Use of 1-(2-naphth-2-ylethyl)-4-(3-trifluoromethylphenyl)-1,2,3,6-tetrahydropyridine and 2-amino-6-trifluoromethoxybenzothiazole or their addition salts with pharmaceutically acceptable acids for the preparation of a medicament intended for the treatment of amyotrophic lateral sclerosis.

8. A pharmaceutical composition in which 1-(2-naphth-2-ylethyl)-4-(3-trifluoromethylphenyl)-1,2,3,6-tetrahydropyridine and 2-amino-6-trifluoromethoxybenzothiazole or their addition salts with pharmaceutically acceptable acids are present as the active principles.

9. A kit intended for the treatment of amyotrophic lateral sclerosis which contains :
a) one or more unit doses of 1-(2-naphth-2-ylethyl)-4-(3-trifluoromethylphenyl)-1,2,3,6-tetrahydropyridine or one of its pharmaceutically acceptable salts, and
b) one or more unit doses of 2-amino-6-trifluoromethoxybenzothiazole or one of its pharmaceutically acceptable salts.

## Patentansprüche

1. Verwendung von 1-(2-Naphth-2-ylethyl)-4-(3-trifluormethylphenyl)-1,2,3,6-tetrahydropyridin oder eines seiner pharmazeutisch annehmbaren Säureadditionssalze zur Herstellung von Medikamenten zur Behandlung von amyotropher Lateralsklerose.

2. Verwendung nach Anspruch 1, worin das Säureadditionssalz das Hydrochlorid ist.

3. Verwendung nach Anspruch 1 oder 2, worin das Medikament eine pharmazeutische Zusammenetzung in Dosiseinheitsform ist.

4. Verwendung nach Anspruch 3, worin die Dosiseinheitsform 0,5 bis 10 mg Wirkstoff enthält.

5. Verwendung nach Anspruch 4, worin die Dosiseinheitsform 1 bis 5 mg Wirkstoff enthält.

6. Verwendung nach einem der Ansprüche 3 bis 5, worin die pharmazeutische Zusammensetzung in Form einer Zusammensetzung für orale Verabreichung vorliegt.

7. Verwendung von 1-(2-Naphth-2-ylethyl)-4-(3-trifluormethylphenyl)-1,2,3,6-tetrahydropyridin und 2-Amino-6-trifluormethoxybenzothiazol oder deren pharmazeutisch annehmbaren Säureadditionssalzen zur Herstellung eines Medikaments zur Behandlung von amyotropher Lateralsklerose.

8. Pharmazeutische Zusammensetzung, die als Wirkstoffe 1-(2-Naphth-2-ylethyl)-4-(3-trifluormethylphenyl)-1,2,3,6-tetrahydropyridin und 2-Amino-6-trifluormethoxybenzothiazol oder deren pharmazeutisch annehmbare Säureadditionssalze enthält.

9. Garnitur zur Behandlung von amyotropher Lateralsklerose enthaltend:
a) eine oder mehrere Einheitsdosen von l-(2-Naphth-2-ylethyl)-4-(3-trifluormethylphenyl)-1,2,3,6-tetrahydropyridin oder eines seiner pharmazeutisch annehmbaren Salze und
b) eine oder mehrere Einheitsdosen von 2-Amino-6-trifluormethoxybenzothiazol oder eines seiner pharmazeutisch annehmbaren Salze.
